# EUROPEAN PATENT SPECIFICATION

(11) **EP 2 163 540 B1**
(45) Date of publication and mention of the grant of the patent: **08.01.2014**
(21) Application number: 08752937.6
(22) Date of filing: 19.05.2008
(51) Int. Cl.: C07C 263/10, C07C 265/04

(54) **METHOD FOR PRODUCING ETHYLENICALLY UNSATURATED GROUP-CONTAINING ISOCYANATE COMPOUND HAVING ETHER BOND**
VERFAHREN ZUR HERSTELLUNG VON ETHYLENISCH UNGESÄTTIGTEM ISOCYANAT MIT ETHERVERBINDUNG
PROCÉDÉ DE PRODUCTION D'UN ISOCYANATE ÉTHYLÉNIQUEMENT INSATURÉ PORTEUR D'UNE LIAISON ÉTHER

(30) Priority: 21.05.2007 JP 2007133935
(43) Date of publication of application: 17.03.2010
(73) Proprietor: Showa Denko K.K., Tokyo 105-8518 (JP)
(72) Inventor: NOZAWA, Kaneo, Tokyo 105-8518 (JP); OHNO, Katsutoshi, Tokyo 105-8518 (JP); HATTORI, Yotaro, Tokyo 105-8518 (JP)
(74) Representative: Strehl Schübel-Hopf & Partner
(86) International application number: PCT/JP2008/059117
(87) International publication number: WO 2008/143207

(56) References cited:
- WO-A1-2007/010996
- WO-A1-2007/020777
- JP-A- 01 052 746

## Description

### Technical Field

The present invention relates to an isocyanate compound having an ether bond and an unsaturated group in its molecule, used in coating materials, UV-curable paints, heat-curable paints, molding materials, adhesives, inks, pressure-sensitive adhesives, resists, optical materials, photo-shaping materials, printing board materials, dental materials, polymer battery materials, and the like.

### Background Art

Reactive resins are used in various fields. Ethylenically unsaturated group-containing isocyanate compounds are useful in producing such resins. The ethylenically unsaturated group-containing isocyanate compounds can react with, for example, functional groups on the main chains of resins, whereby ethylenically unsaturated groups or isocyanate groups are introduced into the resins. Alternatively, the ethylenically unsaturated group-containing isocyanate compounds can react with compounds containing active hydrogen to form various bonds such as urethane bonds, thiourethane bonds, urea bonds and amide bonds, whereby the compounds are converted into reactive monomers having unsaturated groups in their molecules.

In particular, an unsaturated group-containing isocyanate compound having an ether bond in its molecule is expected to be useful for materials having good flexibility. However, any method for efficiently synthesizing the compound has not yet been developed because of the difficulty to synthesizing the compound.

Patent Document 1 discloses a method in which an aminoalcohol having an ether bond is converted into a carbamoyl compound using urea and alcohol, an ester compound is synthesized by the reaction of the carbamoyl compound with an unsaturated carboxylic acid or the chloride thereof, and an ethylenically unsaturated group-containing isocyanate compound having an ether bond is finally produced by thermally decomposing the carbamoyl compound.

A reactive monomer produced by urethanizing the ethylenically unsaturated group-containing isocyanate compound obtained by the method is useful in producing a compound having high flexibility. The method has room for improvement because the carbamoyl compound is thermally decomposed at an extremely high temperature, about 400°C, unsaturated groups are possibly polymerized depending on an apparatus or another factor, and tin contained in a catalyst may have a negative influence on the product.

Patent Document 2 discloses a method in which a specific (poly)amine compound having an ether bond is converted into a corresponding (poly)isocyanate with phosgene.

In this method, phosgene is used to perform the isocyanate-producing reaction and therefore this reaction is very simple. However, this reaction is performed at a high temperature of 100°C to 500°C. Accordingly, the application of an unsaturated compound to the isocyanate-producing reaction possibly causes polymerization and therefore is not preferred.
Patent Document 1: Japanese Patent Laid-Open Publication No. 62-10053
Patent Document 2: Japanese Patent Laid-Open Publication No. 9-216860

### Disclosure of Invention

### <Problems to be Solved by the Invention>

It is an object of the present invention to provide a method for producing an ethylenically unsaturated group-containing isocyanate compound having an ether bond under such conditions that the ether bond is unlikely to be cleaved and the polymerization of an unsaturated group can be suppressed.

### <Means for Solving the Problems>

The inventors have found that a by-product is suppressed from being produced by reaction with hydrogen chloride and the polymerization of an unsaturated group is suppressed in such a manner that a specific reaction solvent, particularly a reaction solvent in which the solubility of hydrogen chloride is 0.1 mole percent or less at 25°C, is used in reaction steps for producing an ethylenically unsaturated group-containing isocyanate compound having an ether bond and reaction is caused within a specific temperature range. This has led to the completion of the present invention. The present invention relates to Items [1] to [11] below.

[1] A method for producing an ethylenically unsaturated group-containing isocyanate compound having an ether bond from an amino alcohol having an ether bond includes using a reaction solvent in which the solubility of hydrogen chloride is 0.1 mole percent or less at 25°C.
[2] The method for producing an ethylenically unsaturated group-containing isocyanate compound having an ether bond as described in the above [1], wherein the reaction solvent is an aromatic or aliphatic hydrocarbon.
[3] The method for producing an ethylenically unsaturated group-containing isocyanate compound having an ether bond as described in the above [1], wherein the reaction solvent is toluene.
[4] The method for producing an ethylenically unsaturated group-containing isocyanate compound having an ether bond as described in the above [1], further including a reaction step performed at a temperature of 0°C to 100°C.
[5] The method for producing an ethylenically unsaturated group-containing isocyanate compound having an ether bond as described in the above [4], wherein the reaction step includes:
   Step (1) of reacting an amino alcohol (I) having an ether bond, represented by Formula (I) below, with hydrogen chloride to produce a compound (III) represented by Formula (III) below;
   Step (2) of reacting the compound (III) with a compound (IV) represented by Formula (IV) or a compound (V) represented by Formula (V) to produce a compound (VI) represented by Formula (VI) below or a compound (VII) represented by Formula (VII) below;
   Step (3) of reacting the compound (VI) or (VII) with phosgene to produce a compound (VIII) represented by Formula (VIII) below or a compound (II) represented by Formula (II) below; and
   Step (4) of contacting the compound (VIII) or (II) with a basic nitrogen compound containing tertiary nitrogen. 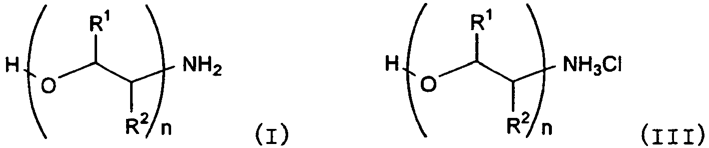
      In Formula (I) or (III), R¹ and R² independently represent a hydrogen atom or a linear or branched alkyl group of 1 to 6 carbon atoms and n represents an integer of 2 to 12. 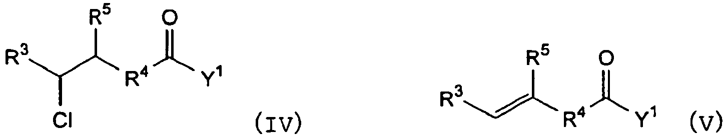
      In Formula (IV) or (V), R³ represents a hydrogen atom, a linear or branched alkyl group of 1 to 6 carbon atoms, or an aryl group; R⁴ represents a single bond, or a linear or branched alkylene group of 1 to 5 carbon atoms; R⁵ represents a hydrogen atom or a methyl group; and Y¹ represents a hydroxy group, a chlorine atom or R⁶O- (where R⁶ represents an alkyl group of 1 to 6 carbon atoms). 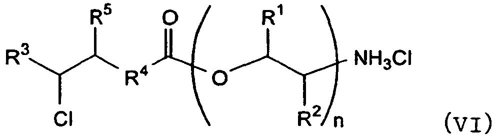 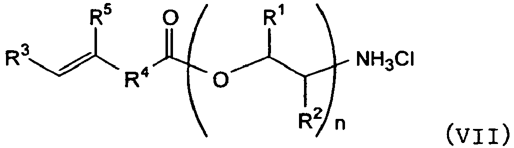
      In Formula (VI) or (VII), R¹, R² and n are the same as R¹, R² and n, respectively in Formula (I) and R³ to R⁵ are the same as R³ to R⁵, respectively, in Formula (IV) or (V). 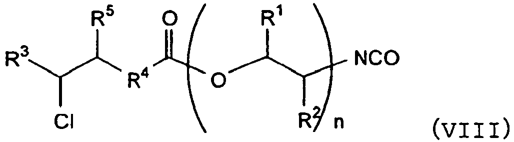 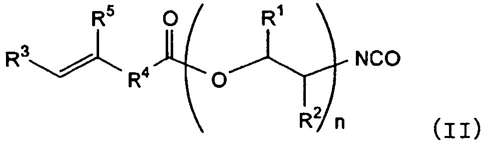
      In Formula (VIII) or (II), R¹, R² and n are the same as R¹, R² and n, respectively, in Formula (I) and R³ to R⁵ are the same as R³ to R⁵, respectively, in Formula (IV) or (V).
[6] The method for producing an ethylenically unsaturated group-containing isocyanate compound having an ether bond as described in the above [5], further including a water-rinsing step of contacting a product obtained in Step (4) with water.
[7] The method for producing an ethylenically unsaturated group-containing isocyanate compound having an ether bond as described in the above [5], wherein the reaction temperature of Step (2) is 65°C to 100°C.
[8] The method for producing an ethylenically unsaturated group-containing isocyanate compound having an ether bond as described in the above [5], wherein Y¹ in the compound (IV) or (V) is a chlorine atom and the reaction of Step (2) is performed at reduced pressure.
[9] The method for producing an ethylenically unsaturated group-containing isocyanate compound having an ether bond as described in the above [8], wherein the reaction of Step (2) is performed in such a manner that an inert gas is introduced into a reaction liquid.
[10] The method for producing an ethylenically unsaturated group-containing isocyanate compound having an ether bond as described in the above [5], wherein the reaction of Step (3) is performed at reduced pressure.
[11] The method for producing an ethylenically unsaturated group-containing isocyanate compound having an ether bond as described in the above [10], wherein the reaction of Step (3) is performed in such a manner that an inert gas is introduced into a reaction liquid.

### <Effect of the Invention>

According to the present invention, a by-product can be suppressed from being produced in the production of an isocyanate by the reaction of an amine hydrochloride having an ether bond with phosgene and an ethylenically unsaturated group-containing isocyanate compound having an ether bond can be safely and readily produced.

### Best Modes for Carrying Out the Invention

The present invention will now be described in detail.

A method for producing an ethylenically unsaturated group-containing isocyanate compound having an ether bond according to the present invention is one for producing the ethylenically unsaturated group-containing isocyanate compound from an amino alcohol having an ether bond and is characterized in that a reaction solvent in which the solubility of hydrogen chloride is 0.1 mole percent or less at 25°C is used. The expression "the solubility of hydrogen chloride is 0.1 mole percent" as used herein means that "0.1 mole of hydrogen chloride is dissolved in one mole of a solvent at a partial pressure of 1 atm".

In view of suppressing the formation of by-products, the solubility of hydrogen chloride is preferably low. The solubility of hydrogen chloride is preferably 0.08 mole percent or less and more preferably 0.06 mole percent or less. The solubility thereof can be determined by a method specified in, for example, "Journal of the American Chemical Society, 1937, Vol. 59, p.p. 1712-1714".

### (A) Solvent

The reaction solvent may be continuously used in all reaction steps, that is, Steps (1), (2), (3) and (4) below. Examples of the solvent include aromatic hydrocarbons such as toluene, xylene, ethylbenzene, mesitylene and cumene and aliphatic hydrocarbons such as pentane, hexane, 2-methylpentane, 3-methylpentane, 2,2-dimethylbutane, heptane, 2,2,4-trimethylpentane, decane, undecane, tetradecane, dodecane, tridecane, cyclopentane, cyclohexane and methylcyclohexane.

Examples of the solubility of gaseous hydrogen chloride in the above solvent are as described below (adapted from "SOLUBILITY DATA SERIES Vol. 42 -HYDROGEN HALIDES IN NON-AQUEOUS SOLVENTS-").

| | |
|---|---|
| Toluene | 0.0425 mole percent |
| Xylene | 0.0575 mole percent |
| Pentane | 0.0047 mole percent |
| Hexane | 0.0112 mole percent |
| Heptane | 0.0147 mole percent |
| 2,2,4-trimethylpentane | 0.0154 mole percent |
| Decane | 0.0298 mole percent |
| Dodecane | 0.0314 mole percent |
| Cyclohexane | 0.0154 mole percent |

In particular, the aromatic hydrocarbons are preferred and toluene is more preferred because of the low solubility of hydrogen chloride and ease in handling. These solvents are effective in suppressing the cleavage of ether bonds and the addition of hydrogen chloride to unsaturated groups or NCO groups. This is probably because the solubility of gaseous hydrogen chloride in these solvents is low.

### (B) Reaction temperature

In the present invention, the temperature of reaction is preferably 0°C to 100°C and more preferably 10°C to 100°C through all steps for producing the ethylenically unsaturated group-containing isocyanate compound having an ether bond from the amino alcohol having an ether bond.

Temperatures higher than the above temperature range may cause a reduction in yield in some cases because of unexpected side reactions such as the polymerization of unsaturated groups, the addition of hydrogen chloride to unsaturated groups, and the cleavage of ether bonds by hydrogen chloride. In contrast, temperatures lower than the temperature range tends to cause a reduction in conversion.

In the temperature range, more preferred conditions are as described below. In the case where the reaction temperature is set to a relatively high value within the temperature range, the cleavage of ether bonds and the addition of hydrogen chloride to unsaturated groups or NCO groups tend to be suppressed. This is probably because the concentration of hydrogen chloride is low under high temperature conditions.

### (C) Reaction steps

Reaction steps for producing the ethylenically unsaturated group-containing isocyanate compound having an ether bond are described below in detail. In descriptions below, a compound represented by Formula (I) is sometimes denoted as "compound (I)" and the same applies to other compounds represented by other formulas.

In order to prevent polymerization, the reaction steps are preferably performed in the presence of a polymerization inhibitor. The polymerization inhibitor may be a phenolic antioxidant, phenothiazine or derivatives thereof, a stable free-radical compound, or the like. Examples of the polymerization inhibitor include phenolic antioxidants such as 2,6-di-t-butyl-4-methylphenol, 2,4,6-tri-t-butylphenol, and 2,2'-methylenebis-(4-methyl-6-t-butylphenol); phenothiazine or derivatives thereof such as phenothiazine and styrenated phenothiazine; and stable free-radical compounds such as 2,2,6,6-tetramethylpiperidinooxyl and 4-hydroxy-2,2,6,6-tetramethylpiperidinooxyl.

### [Step (1)]

Step (1) in the method of the present invention is a step for producing a hydroxylamine hydrochloride compound represented by Formula (III) below from an amino alcohol represented by Formula (I) below and hydrogen chloride.

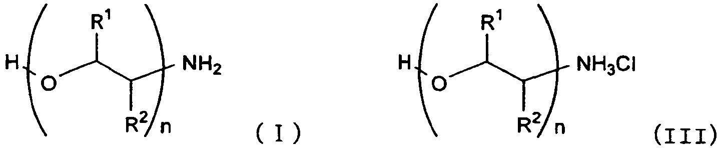

In Formula (I) or (III), R¹ and R² independently represent a hydrogen atom or a linear or branched alkyl group of 1 to 6 carbon atoms and n represents an integer of 2 to 12.

### [Step (2)]

Step (2) in the present invention is a step for producing an ester compound represented by Formula (VI) or (VII) below from the hydroxylamine hydrochloride compound (III) and a compound represented by Formula (IV) or (V) below.

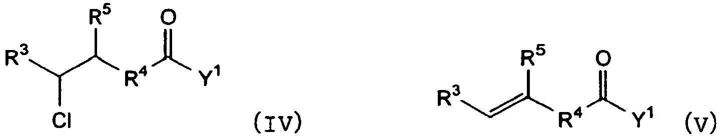

In Formula (IV) or (V), R³ represents a hydrogen atom, a linear or branched alkyl of 1 to 6 carbon atoms or an aryl group; R⁴ represents a single bond or a linear or branched alkylene group of 1 to 5 carbon atoms; R⁵ represents a hydrogen atom or a methyl group; and Y¹ represents a hydroxy group, a chlorine atom or R⁶O- (where R⁶ represents an alkyl group of 1 to 6 carbon atoms).

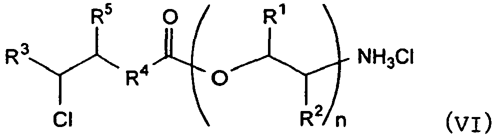

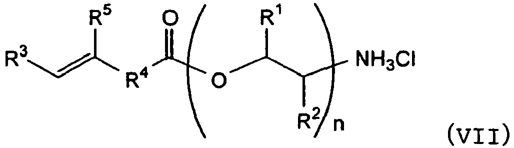

In Formula (VI) or (VII), R¹, R² and n are the same as R¹, R² and n, respectively, in Formula (I) and R³ to R⁵ are the same as R³ to R⁵, respectively, in Formula (IV) or (V).

### [Step (3)]

Step (3) in the method of the present invention is a step for producing an isocyanate compound from the ester compound (VI) or (VII) and phosgene. Step (3) is hereinafter referred to as "Step (3a)" or "Step (3b)" in the case where the isocyanate compound is derived from the compound (VI) or (VII), respectively.

### (Step (3a))

Step (3a) in the method of the present invention is a step for producing an isocyanate compound represented by Formula (VIII) below from the ester compound (VI) and phosgene.

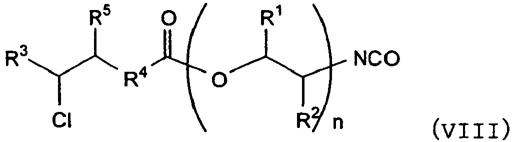

In Formula (VIII), R¹, R² and n are the same as R¹, R² and n, respectively, in Formula (I) and R³ to R⁵ are the same as R³ to R⁵, respectively, in Formula (IV).

### (Step (3b))

Step (3b) in the method of the present invention is a step for producing an isocyanate compound having an ether bond represented by Formula (II) below from the ester compound (VII) and phosgene.

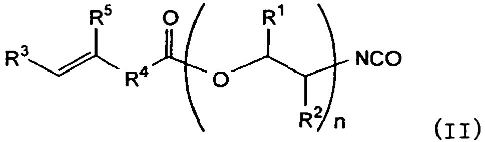

In Formula (II), R¹, R² and n are the same as R¹, R² and n, respectively, in Formula (I) and R³ to R⁵ are the same as R³ to R⁵, respectively, in Formula (V).

### [Step (4)]

Step (4) in the method of the present invention is a step for contacting the isocyanate compound (VIII) or (II) with a basic nitrogen compound containing tertiary nitrogen. Step (4) is hereinafter referred to as "Step (4a)" or "Step (4b)" in the case where the compound (VIII) or (II), respectively is derived.

The above steps are described below in detail.

### <Step (1)>

The amino alcohol (I), which is used in Step (1), is not particularly limited. Examples thereof include 2-(2-aminoethoxy)ethanol, 2-methyl-2-(2-amino-2-methylethoxy)ethanol, 2-(2-amino-1-methylethoxy)-1-methyl-ethanol, (2-amino-2-methyl-1-methylethoxy)-1-methyl-2-methylethanol and 2-(2-(2-aminoethoxy)ethoxy)ethanol. In particular, 2-(2-aminoethoxy)ethanol is preferred.

The reaction temperature of Step (1) depends on the type of a compound used. The reaction temperature thereof is preferably 0°C to 100°C, more preferably 15°C to 100°C, and further more preferably 30°C to 100°C. An excessive reduction in the reaction temperature thereof possibly reduces the rate of reaction. In contrast, an excessive increase in the reaction temperature thereof possibly causes a produced salt to be thermally decomposed.

The amount of the solvent used is adjusted such that the amount of the amino alcohol (I) is preferably 1 to 50% by mass, more preferably 2 to 30% by mass, and further more preferably 5 to 20% by mass with respect to the total amount of the amino alcohol (I), hydrogen chloride and the solvent. A reduction in the amount of the solvent used possibly causes insufficient mixing during reaction to reduce the reaction rate. In contrast, an increase in the amount of the solvent used does not affect the reaction but possibly increases the amount of solvent waste to increase the impact on the environment.

The amine hydrochloride compound (III), which is obtained in Step (1), can be purified by a common process such as extraction or recrystallization and can be used in Step (2) without being purified.

### <Step (2)>

The compound (IV), which is used in Step (2), is not particularly limited and is commercially available. Examples of the compound (IV) include 3-chloropropionic acid, 3-chlorobutyric acid, 4-chlorobutyric acid, 3-chloro-2-methylpropionic acid, 4-chlorovaleric acid, 3-chlorovaleric acid, 4-chloro-3-methylbutyric acid, 3-chloro-3-methylbutyric acid, 3-chloro-3-phenylpropionic acid, 3-chloro-3-phenyl-2-methyl propionic acid, chlorides of these carboxylic acids, and esters derived from these carboxylic acids and linear or branched alcohols of 1 to 6 carbon atoms. In the method of the present invention, the compound (IV) is preferably a carboxylic acid chloride (Y¹ is a chlorine atom) and more preferably 3-chloro-2-methylpropionyl chloride or 3-chloropropionyl chloride.

The compound (V) is not particularly limited and is commercially available. Examples of the compound (V) include acrylic acid, methacrylic acid, 3-methyl-3-butenonic acid, tiglic acid, 4-methyl-4-pentenoic acid, α-methylcinnamic acid, chlorides of these carboxylic acids, and esters derived from these carboxylic acids and linear or branched alcohols of 1 to 6 carbon atoms. In the method of the present invention, the compound (V) is preferably a carboxylic acid chloride (Y¹ is a chlorine atom) and more preferably methacryloyl chloride or acryloyl chloride.

The reaction temperature of Step (2) depends on the type of a compound used. The reaction temperature thereof is preferably 65°C to 100°C and more preferably 70°C to 95°C. An excessive reduction in the reaction temperature thereof possibly reduces the rate of reaction. An excessive increase in the reaction temperature thereof possibly causes the thermal decomposition of a salt produced in Step (1) and causes the cleavage of an ether bond due to gaseous hydrogen chloride produced in Step (2), thereby causing a reduction in yield. In particular, the compound (V) is thermally dehydrochlorinated and therefore unsaturated bonds produced thereby are possibly polymerized.

In the present invention, the reaction temperature of Step (2) significantly affects the total yield of reaction and the amount of impurities produced. In particular, the yield increases with the increase of the reaction temperature from 65°C to 100°C.

The document "Journal of Organic Chemistry, 1981, 46, 3361-3364" reports that about 50% of tetrahydrofuran heated at 100°C for eight hours in the presence of hydrogen chloride decomposes. In the present invention, although the reaction is performed at a temperature substantially equal to that reported in this document, no ether bond is cleaved. This is probably because the solubility of hydrogen chloride in tetrahydrofuran reported in this document is about 50 times the solubility of hydrogen chloride in a solvent, such as toluene, used herein.

The present invention is characterized in that the reaction solvent, in which the solubility of hydrogen chloride is 0.1 mole percent or less at 25°C, is used in all steps and thereby reaction is performed with the concentration of hydrogen chloride in a reaction system maintained low. When the compound (IV) or (V), which is used in the second step, is a carboxylic acid chloride (Y¹ is a chlorine atom), an equimolecular amount of hydrogen chloride is produced; hence, the reaction of the second step is preferably performed at reduced pressure. The reaction may be performed under reduced pressure conditions, for example, at a slightly reduced pressure of 700 to 750 torr. Since the reaction is performed at such a reduced pressure, hydrogen chloride, which is by-produced during the reaction, can be efficiently removed. The reaction may be performed in such a manner that a reaction liquid is bubbled with an inert gas such as nitrogen.

The amount of the compound (IV) or (V) used with respect to the amine hydrochloride compound (III) depends on the type thereof. The amount of the compound (IV) or (V) used is preferably 0.5 to 10 moles and more preferably 0.8 to 5 moles per mole of the amine hydrochloride compound (III). A reduction in the amount of the compound (IV) or (V) used possibly causes a reduction in yield and an increase in the amount of impurities. An increase in the amount of the compound (IV) or (V) used possibly causes a reduction in yield and an increase in the amount of wastes to increase the impact on the environment.

The ester compound (VI) or (VII), which is produced in Step (2), can be purified by a common process such as extraction, recrystallization or distillation and can be used in Step (3) without being purified.

The amount of the solvent used is adjusted such that the amount of the hydroxyamine hydrochloride compound (III) is preferably 1 to 50% by mass, more preferably 2 to 30% by mass, and further more preferably 5 to 20% by mass with respect to the total amount of the hydroxyamine hydrochloride compound (III), the compound (IV) or (V) and the solvent. A reduction in the amount of the solvent used possibly causes insufficient mixing during reaction to reduce the reaction rate. In contrast, an increase in the amount of the solvent used does not affect the reaction but possibly increases the amount of solvent waste to increase the impact on the environment.

### <Step (3a)>

The reaction temperature of Step (3a) depends on the type of a compound used. The reaction temperature thereof is preferably 65°C to 100°C and more preferably 70°C to 95°C. An excessive reduction in the reaction temperature thereof possibly reduces the reaction rate. An excessive increase in the reaction temperature thereof possibly causes thermal dehydrochlorination; hence, unsaturated bonds produced thereby may be polymerized and an ether bond may be cleaved due to gaseous hydrogen chloride produced, thereby causing a reduction in yield.

The ester compound (VI) reacts with phosgene at a molar ratio of 1:1 theoretically. In order to smoothly perform this reaction, an excessive amount of phosgene is preferably used. The amount of phosgene used with respect to the ester compound (VI) depends on the type thereof. The amount of phosgene used is preferably 1 to 10 moles and more preferably 1 to 5 moles per mole of the ester compound (VI). A reduction in the amount of phosgene used possibly causes a reduction in yield and an increase in the amount of impurities because the ester compound (VI) remains without reacting. An increase in the amount of phosgene used does not affect the reaction but possibly causes the need for a special detoxifying apparatus or the like and an increase in the impact on the environment.

In the method of the present invention, the reaction of Step (3a) is preferably performed at reduced pressure. The reaction may be performed under reduced pressure conditions, for example, at a slightly reduced pressure of 700 to 750 torr. Since the reaction is performed at such a reduced pressure, hydrogen chloride, which is by-produced during the reaction, can be efficiently removed. The reaction may be performed in such a manner that a reaction liquid is bubbled with an inert gas such as nitrogen.

The isocyanate compound (VIII), which is obtained in Step (3a), can be purified by a common process such as extraction, recrystallization or distillation and can be used in Step (4a) without being purified.

The amount of the solvent used is adjusted such that the amount of the ester compound (IV) is preferably 0.5 to 80% by mass and more preferably 5 to 50% by mass with respect to the total amount of the ester compound (IV), phosgene and the solvent. A reduction in the amount of the solvent used possibly causes insufficient mixing during the reaction to reduce the reaction rate. In contrast, an increase in the amount of the solvent used does not affect the reaction but possibly increases the amount of solvent waste to increase the impact on the environment.

### <Step (4a)>

Step (4a) in the method of the present invention is a step for producing the unsaturated group-containing isocyanate compound having an ether bond, which is represented by Formula (II), by dehydrochlorinating the isocyanate compound (VIII) in the presence of the basic nitrogen compound.

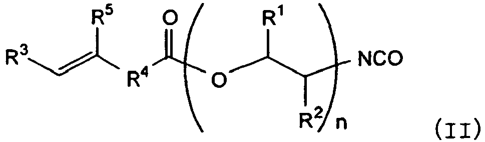

In Formula (II), R¹, R² and n are the same as R¹, R² and n, respectively, in Formula (I) and R³ to R⁵ are the same as R³ to R⁵, respectively, in Formula (IV).

The reaction temperature of Step (4a) depends on the type of a compound used. The reaction temperature thereof is preferably 65°C to 100°C and more preferably 70°C to 95°C. An excessive reduction in the reaction temperature thereof possibly reduces the reaction rate. An excessive increase in the reaction temperature thereof possibly causes thermal dehydrochlorination; hence, unsaturated bonds produced thereby may be polymerized and the ether bond may be cleaved due to gaseous hydrogen chloride produced, thereby causing a reduction in yield.

The basic nitrogen compound, which is used in Step (4a), may be a compound containing a basic nitrogen atom. If the basic nitrogen atom is bonded to a hydrogen atom, the basic nitrogen compound reacts with an isocyanate group present in the isocyanate compound (VII). This possibly causes a reduction in yield. Therefore, the basic nitrogen compound preferably contains tertiary nitrogen.

In Step (4a), an ethylenically unsaturated bond is introduced into a molecule by dehydrochlorination. A weakly basic nitrogen compound, such as quinoline, having an aromatic ring containing a nitrogen atom is insufficient to efficiently perform dehydrochlorination; hence, a compound with relatively high basicity needs to be used. Therefore, the basic nitrogen compound, which contains tertiary nitrogen, preferably has a substituent other than an aromatic ring, such as an alkyl group, bonded to the tertiary nitrogen atom and the tertiary nitrogen atom is more preferably bonded to one or no aromatic ring.

Examples of the basic nitrogen compound include trimethylamine, triethylamine, tripropylamine, tributylamine, tripentylamine and tetramethylenediamine. These basic nitrogen compound may be used alone or in combination.

The amount of the basic nitrogen compound used depends on the type thereof. The amount of the basic nitrogen compound used is preferably 0.5 to 10 moles, more preferably 0.8 to 5.0 moles, and further more preferably 0.9 to 2.0 moles per mole of alkali-decomposable chlorine present in a reaction liquid at the termination of the reaction in Step (3a). A reduction in the amount of the basic nitrogen compound used possibly causes a reduction in yield. An increase in the amount of the basic nitrogen compound used possibly causes a reduction in the stability of the ethylenically unsaturated group-containing isocyanate compound (II) and an increase in production cost.

The amount of the alkali-decomposable chlorine therein is determined in such a manner that the reaction liquid obtained in Step (3) is diluted with a solvent mixture of methanol and water, an aqueous solution of sodium hydroxide is added to the diluted reaction liquid, this mixture is heated and then measured by potentiometric titration using a silver nitrate solution.

The amount of the solvent used is adjusted such that the amount of the isocyanate compound (VIII) is preferably 0.1 to 80% by mass and more preferably 1 to 50% by mass with respect to the total amount of the isocyanate compound (VIII), the basic nitrogen compound and the solvent. A reduction in the amount of the solvent used possibly causes insufficient mixing during the reaction to reduce the reaction rate. In contrast, an increase in the amount of the solvent used does not affect the reaction but possibly increases the amount of solvent waste to increase the impact on the environment.

### <Step (3b)>

The reaction temperature of Step (3b) depends on the type of a compound used. The reaction temperature thereof is preferable 65°C to 100°C and more preferably 70 to 95°C. An excessive reduction in the reaction temperature thereof possibly reduces the reaction rate. An excessive increase in the reaction temperature thereof possibly causes thermal dehydrochlorination; hence, unsaturated bonds produced thereby may be polymerized and an ether bond may be cleaved due to gaseous hydrogen chloride produced, thereby causing a reduction in yield.

The ester compound (VII) reacts with phosgene at a molar ratio of 1:1 theoretically. In order to smoothly perform this reaction, an excessive amount of phosgene is preferably used. The amount of phosgene used with respect to the ester compound (VII) depends on the type thereof. The amount of phosgene used is preferably 1 to 10 moles and more preferably 1 to 5 moles per mole of the ester compound (VII). A reduction in the amount of phosgene used possibly causes a reduction in yield and an increase in the amount of impurities because the ester compound (VII) remains without reacting. An increase in the amount of phosgene used does not affect the reaction but possibly causes the need for a special detoxifying apparatus or the like and an increase in the impact on the environment.

In the method of the present invention, the reaction of Step (3b) is preferably performed at reduced pressure. The reaction may be performed under reduced pressure conditions, for example, at a slightly reduced pressure of 700 to 750 torr. Since the reaction is performed at such a reduced pressure, hydrogen chloride, which is by-produced during the reaction, can be efficiently removed. The reaction may be performed in such a manner that a reaction liquid is bubbled with an inert gas such as nitrogen.

The amount of the solvent used is adjusted such that the amount of the ester compound (VII) is preferably 0.5 to 80% by mass and more preferably 5 to 50% by mass with respect to the total amount of the ester compound (VII), phosgene and the solvent. A reduction in the amount of the solvent used possibly causes insufficient mixing during the reaction to reduce the reaction rate. In contrast, an increase in the amount of the solvent used does not affect the reaction but possibly increases the amount of solvent waste to increase the impact on the environment.

### <Step (4b)>

In the method of the present invention, the following step is preferably performed: Step (4b) of contacting the isocyanate compound (II), which is obtained in Step (3b), with the basic nitrogen compound, which contains tertiary nitrogen.

The amount of the basic nitrogen compound used depends on the type of a compound used. The amount of the basic nitrogen compound used is preferably 0.5 to 10 moles, more preferably 0.8 to 5.0 moles, and further more preferably 0.9 to 2.0 moles per mole of alkali-decomposable chlorine present in a reaction liquid at the termination of the reaction in Step (3b). A reduction in the amount of the basic nitrogen compound used possibly causes a reduction in yield. An increase in the amount of the basic nitrogen compound used possibly causes a reduction in the stability of the ethylenically unsaturated group-containing isocyanate compound (II) and an increase in production cost.

The amount of the alkali-decomposable chlorine therein is determined in such a manner that the reaction liquid obtained in Step (3b) is diluted with a solvent mixture of methanol and water, an aqueous solution of sodium hydroxide is added to the diluted reaction liquid, this mixture is heated and then measured by potentiometric titration using a silver nitrate solution.

The amount of the solvent used is adjusted such that the amount of the ethylenically unsaturated group-containing isocyanate compound (II) is preferably 0.1 to 80% by mass and more preferably 1 to 50% by mass with respect to the total amount of the ethylenically unsaturated group-containing isocyanate compound (II), the basic nitrogen compound and the solvent. A reduction in the amount of the solvent used possibly causes insufficient mixing during the reaction to reduce the reaction rate. In contrast, an increase in the amount of the solvent used does not affect the reaction but possibly increases the amount of solvent waste to increase the impact on the environment.

### (D) Water-rinsing step

The following step is preferably performed subsequently to the above reaction steps: a water-rinsing step of contacting a product obtained in Step (4a) or (4b) with water. Some of isocyanate compounds react with water to decompose. However, the ethylenically unsaturated group-containing isocyanate compound (II), which is produced in Step (4a) or (4b), does not decompose if the ethylenically unsaturated group-containing isocyanate compound is contacted with water. Therefore, an amine hydrochloride and the like can be efficiently removed from the reaction liquid in the water-rinsing step.

### (E) Purification step

The ethylenically unsaturated group-containing isocyanate compound (II) can be purified by a common process such as filtration, extraction, recrystallization or distillation. A process and apparatus for purifying the compound (II) is not particularly limited. The process may be simple distillation or rectification. For simple distillation, a common batch distillation unit or a thin-film distillation unit can be used. For rectification, a distillation unit including a rectifying column and a reflux system can be used. The temperature of distillation is preferably low because unnecessary heat history can be avoided. For particular distillation conditions, the temperature in a still or the temperature of a heat transfer surface is preferably 150°C or lower and more preferably 130°C or lower.

### Examples

The present invention will now be further described in detail with reference to examples. The present invention is not limited to the examples. Analyzers and analysis conditions used in the examples are as described below.

### <Gas chromatography (GC)>

Analyzer: Agilent Technologies 6850
Column: DB-1 (manufactured by J&W) having a length of 30 m, an inner diameter of 0.32 mm, and a film thickness of 1 µm
Column temperature: Heated to from 50°C to 300°C at a rate of 10 °C/min and held at 300°C for five minutes
Integrator: Chemistation made by Agilent Technologies
Injection temperature: 250°C
Detector temperature: 250°C, FID
Detector: FID with a H₂ flow rate of 40 mL/min and an air flow rate of 450 mL/min
Carrier gas: He at a flow rate of 10 mL/min

### <Automatic titrator>

Analyzer: COM-550 manufactured by Hiranuma Sangyo Co., Ltd.

### <Method for determining alkali-decomposable chlorine>

Into a 300-ml stoppered conical flask, 0.5 g of a sample was precisely weighed. Into the conical flask, 100 ml of a methanol-purified water mixture (a volume ratio of 70:30) and then 10 ml of a 30% aqueous solution of sodium hydroxide were added. A cooling tube was attached to the conical flask. After being heated at 80°C for one hour in a water bath under reflux, the conical flask was cooled to room temperature. The solution thereby obtained was taken into a 200-ml beaker. To the obtained solution, 100 ml of purified water and then 1 ml of (1 + 1) nitric acid were added. The concentration of alkali-decomposable chlorine in this mixture was determined by potentiometric titration using a 1/50 normality silver nitrate solution. A potentiometric titrator ("COM-550" manufactured by Hiranuma Sangyo Co., Ltd.) was used herein.

### [Example 1]

### <Step (1)>

Into a 500-mL four-neck flask including an agitator, a thermometer, a dropping funnel and a reflux cooler, 35.0 g (0.33 mol) of 2-(2-aminoethoxy)ethanol and 350 mL of toluene (in which the solubility of gaseous hydrogen chloride is 0.0425 mole percent at 25°C and a partial pressure of 1 atm as given in *"*SOLUBILITY DATA SERIES Vol. 42 -HYDROGEN HALIDES IN NON-AQUEOUS SOLVENTS*-")* were charged under a nitrogen atmosphere. The flask was heated to 30°C, whereby 2-(2-aminoethoxy)ethanol was melted. Gaseous hydrogen chloride was supplied to the flask for one hour at a flow rate of 150 mL/min at a temperature of 75°C to 90°C.

### <Step (2)>

A reaction liquid obtained in Step (1) was heated to 80°C. To the reaction liquid, 0.2 g of phenothiazine was added and 40.0 g (0.38 mol) of methacryloyl chloride was then supplied in 1.5 hours, followed by heating at 80°C for 0.5 hour.

### <Step (3)>

A reaction liquid obtained in Step (2) was kept at 85°C. To this reaction liquid, 0.2 g of phenothiazine was added and 53.7 g (0.55 mol) of phosgene was then supplied in five hours, followed by heating at 85°C for one hour. The phosgene dissolved in this reaction liquid was removed by introducing nitrogen into this reaction liquid. The analysis of this resulting reaction liquid by gas chromatography showed that 39.7 g (0.20 mol) of 2-(isocyanatoethyloxy)ethyl methacrylate (hereinafter referred to as "MOI-EG") was obtained and the yield thereof was 59.8% (on the basis of 2-(2-aminoethoxy)ethanol).

### <Purification step>

A solvent was distilled off from this reaction liquid at a reduced pressure of 5 kPa with a vacuum pump. The condensed liquid was poured into a 100-mL flask, 39.5 g of phenothiazine was added to the flask, and the condensed liquid was subjected to distillation at a reduced pressure of 0.1 kPa, whereby a 91-95°C fraction was obtained. This showed that 35.8 g (0.18 mol) of MOI-EG was obtained and the yield thereof was 53.0% (on the basis of 2-(2-aminoethoxy)ethanol).

### [Example 2]

### <Step (1)>

Substantially the same operation as that performed in Step (1) of Example 1 was performed.

### <Step (2)>

Each liquid obtained in Step (1) was heated to a temperature (X°C) shown in Table 1. To the liquid, 0.2 g of phenothiazine was added and 40.0 g (0.38 mol) of methacryloyl chloride was then supplied in 1.5 hours, followed by heating at X°C for 0.5 hour.

### <Step (3)>

A liquid obtained in Step (2) was kept at 85°C. To this liquid, 0.2 g of phenothiazine was added and 53.7 g (0.55 mol) of phosgene was then supplied in five hours, followed by heating at 85°C for one hour. The phosgene dissolved in this liquid was removed by introducing nitrogen into this liquid. The analysis of this resulting liquid by gas chromatography showed that MOI-EG was obtained at a yield of Y% as shown in Table 1 (on the basis of 2-(2-aminoethoxy)ethanol). The yield of a by-product formed by the addition of hydrogen chloride (HCl) to an unsaturated group in MOI-EG was Z% as shown in Table 1. The relationship between X, Y and Z is shown in Table 1.

**[Table 1]**

| Reaction temperature (X°C) | 65°C | 75°C | 80°C | 90°C | 100°C |
|---|---|---|---|---|---|
| Yield of MOI-EG (Y%) | 47.2% | 59.1% | 59.8% | 67.0% | 68.5% |
| Yield of by-product (Z%) | 8.1% | 10.8% | 9.8% | 8.2% | 5.5% |

Example 2 shows that the increase of the temperature of the esterification reaction in Step (2) from 65°C to 100°C increases the yield of a target product obtained in Step (3) and the increase from 75°C to 100°C reduces the amount of the by-product, that is, a hydrogen chloride adduct produced. An increase in final yield is relatively low at 100°C. This suggests that an ether bond was cleaved and/or a high-boiling point compound was produced. An increase in reaction temperature provides better results; however, an excessive increase in reaction temperature possibly causes the polymerization of unsaturated groups. Therefore, the reaction temperature of Step (2) is preferably 70°C to 95°C.

### [Example 3]

### <Step (1)>

Into a 500-mL four-neck flask including an agitator, a thermometer, a dropping funnel and a reflux cooler, 20.0 g (0.19 mol) of 2-(2-aminoethoxy)ethanol and 200 mL of toluene (in which the solubility of gaseous hydrogen chloride is 0.0425 mole percent at 25°C and a partial pressure of 1 atm as given in *"*SOLUBILITY DATA SERIES Vol. 42 -HYDROGEN HALIDES IN NON-AQUEOUS SOLVENTS*-")* were charged under a nitrogen atmosphere. The flask was heated to 25°C, whereby 2-(2-aminoethoxy) ethanol was melted. Gaseous hydrogen chloride was supplied to the flask for one hour at a flow rate of 150 mL/min at a temperature of 75°C to 90°C.

### <Step (2)>

To a reaction liquid obtained in Step (1), 0.2 g of phenothiazine was added. To the resulting reaction liquid, 23.9 g (0.23 mol) of methacryloyl chloride was supplied at an internal temperature of 85°C in 1.7 hours in such a manner that the pressure in a system was maintained at 720 torr and the resulting reaction liquid was bubbled with N₂ at a flow rate of 10 mL/min, followed by heating at 85°C for 5.0 hours.

### <Step (3)>

A reaction liquid obtained in Step (2) was kept at 90°C. To this reaction liquid, 0.2 g of phenothiazine was added and 34.0 g (0.34 mol) of phosgene was then supplied in five hours, followed by heating at 90°C for one hour. The phosgene dissolved in this reaction liquid was removed by introducing nitrogen into this reaction liquid. The analysis of this resulting reaction liquid by gas chromatography showed that 27.7 g (0.14 mol) of MOI-EG was obtained and the yield thereof was 73.3% (on the basis of 2-(2-aminoethoxy)ethanol). The yield of a by-product formed by the addition of hydrogen chloride (HCl) to an unsaturated group in MOI-EG was 3.2% (on the basis of 2-(2-aminoethoxy)ethanol).

### [Example 4]

### <Step (1)>

Into a 500-mL four-neck flask including an agitator, a thermometer, a dropping funnel and a reflux cooler, 20.0 g (0.19 mol) of 2-(2-aminoethoxy)ethanol and 200 mL of toluene (in which the solubility of gaseous hydrogen chloride is 0.0425 mole percent at 25°C and a partial pressure of 1 atm as given in *"*SOLUBILITY DATA SERIES Vol. 42 -HYDROGEN HALIDES IN NON-AQUEOUS SOLVENTS*-")* were charged under a nitrogen atmosphere. The flask was heated to 30°C, whereby 2-(2-aminoethoxy)ethanol was melted. Gaseous hydrogen chloride was supplied to the flask for one hour at a flow rate of 150 mL/min at a temperature of 75°C to 90°C.

### <Step (2)>

A reaction liquid obtained in Step (1) was heated to 95°C. To this reaction liquid, 0.2 g of phenothiazine was added and 21.9 g (0.21 mol) of methacryloyl chloride was then supplied in 1.0 hour, followed by heating at 95°C for 3.0 hours.

### <Step (3)>

A reaction liquid obtained in Step (2) was kept at 90°C. To this reaction liquid, 0.2 g of phenothiazine was added and 34.0 g (0.34 mol) of phosgene was then supplied in five hours, followed by heating at 90°C for one hour. The phosgene dissolved in this reaction liquid was removed by introducing nitrogen into this reaction liquid, whereby 210.7 g of a reaction liquid was obtained. The analysis of this reaction liquid by gas chromatography showed that MOI-EG was obtained at a yield of 73.4% (on the basis of 2-(2-aminoethoxy)ethanol). A solvent was distilled off from this reaction liquid at an internal temperature of 65°C and a pressure of 10 to 12 kPa, whereby the concentration of toluene therein was reduced to 20%.

### <Step (4)>

Into a 1000-mL four-neck flask including an agitator, a thermometer, a dropping funnel and a reflux cooler, 500 g of the condensed reaction liquid obtained in Step (3) (294.0 g of MOI-EG) and 3 g of phenothiazine were charged. The analysis of alkali-decomposable chlorine in the condensed reaction liquid showed that the amount of alkali-decomposable chlorine in 500 g of the condensed reaction liquid was 0.83 mol. To the reaction liquid, 84.1 g of (0.83 mol) of triethylamine was supplied at an internal temperature of 60°C in 120 minutes, followed by heating at 90°C for five hours. After the reaction liquid was cooled to room temperature, triethylamine hydrochloride was removed from the reaction liquid by filtration and the reaction liquid was then rinsed with toluene. The reaction liquid was condensed at an internal temperature of 65°C and a pressure of 10 to 12 kPa, whereby 478.0 g of a condensed toluene solution containing 293.6 g of MOI-EG was obtained.

### <Water-rinsing step>

Into a 2000-mL four-neck flask including an agitator, a thermometer, a dropping funnel and a reflux cooler, 300.0 g of the condensed toluene solution obtained in Step (4) (184.3 g of MOI-EG) and 1500 mL of methylene chloride were charged. The internal temperature of the flask was kept at 10°C and 600 mL of water was supplied to the flask, followed by agitation for 30 minutes. The flask was kept stationary for ten minutes and the lower one of two layers in the flask was taken out, whereby 2300 g of a methylene chloride solution was obtained. The methylene chloride solution was condensed at atmospheric pressure and methylene chloride was distilled off from the solution at a pressure of 10 to 13 kPa, whereby 312 g of a condensed solution containing 184.1 g of MOI-EG was obtained. The condensed solution was purified with a thin-film distillation unit, whereby 153.4 g of MOI-EG was obtained. The analysis of MOI-EG by gas chromatography confirmed that the yield of MOI-EG was 80.0%.

### [Example 5]

### <Step (1)>

Into a 500-mL four-neck flask including an agitator, a thermometer, a dropping funnel and a reflux cooler, 20.0 g (0.19 mol) of 2-(2-aminoethoxy)ethanol and 200 mL of toluene (in which the solubility of gaseous hydrogen chloride is 0.0425 mole percent at 25°C and a partial pressure of 1 atm as given in *"*SOLUBILITY DATA SERIES Vol. 42 -HYDROGEN HALIDES IN NON-AQUEOUS SOLVENTS*-")* were charged under a nitrogen atmosphere. The flask was heated to 30°C, whereby 2-(2-aminoethoxy)ethanol was melted. Gaseous hydrogen chloride was supplied to the flask for one hour at a flow rate of 150 mL/min at a temperature of 75°C to 90°C.

### <Step (2)>

A reaction liquid obtained in Step (1) was heated to 95°C. To this reaction liquid, 0.2 g of phenothiazine was added and 26.5 g (0.21 mol) of 3-chloropropionyl chloride was then supplied in 1.0 hour, followed by heating at 95°C for 3.0 hours.

### <Step (3)>

A reaction liquid obtained in Step (2) was kept at 90°C. To this reaction liquid, 0.2 g of phenothiazine was added and 34.0 g (0.34 mol) of phosgene was then supplied in five hours, followed by heating at 90°C for one hour. The phosgene dissolved in this reaction liquid was removed by introducing nitrogen into this reaction liquid, whereby 207.1 g of a reaction liquid was obtained. The concentration of alkali-decomposable chlorine in this reaction liquid was determined to be 4.0%.

### <Step (4)>

To the reaction liquid obtained in Step (3), 0.2 g of phenothiazine was added. The internal temperature of the resulting reaction liquid was adjusted to 60°C and 22.1 g (0.22 mol) of triethylamine was added dropwise to the resulting reaction liquid, followed by heating for 8.0 hours. After the reaction liquid was cooled to room temperature, triethylamine hydrochloride was removed from the reaction liquid by filtration and the reaction liquid was then rinsed with toluene. To the reaction liquid, 0.2 g of phenothiazine was added. The resulting reaction liquid was condensed at an internal temperature of 65°C and a pressure of 10 to 12 kPa, whereby 36.7 g of a condensed toluene solution was obtained. The analysis of the condensed toluene solution by gas chromatography confirmed that 24.8 g of 2-(isocyanatoethyloxy)ethyl acrylate (hereinafter referred to as "AOI-EG") was obtained and the yield thereof was 70.4% (on the basis of 2-(2-aminoethoxy)ethanol).

### <Water-rinsing step>

Into a 500-mL four-neck flask including an agitator, a thermometer, a dropping funnel and a reflux cooler, 36.7 g of the condensed toluene solution obtained in Step (4) (24.8 g of AOI-EG) and 165 mL of methylene chloride were charged. The internal temperature of the flask was kept at 10°C and 66 mL of water was supplied to the flask, followed by agitation for 30 minutes. The flask was kept stationary for ten minutes and the lower one of two layers in the flask was taken out, whereby 247 g of a methylene chloride solution was obtained. The methylene chloride solution was condensed at atmospheric pressure and methylene chloride was distilled off from the solution at a pressure of 10 to 13 kPa, whereby 37.8 g of a condensed solution was obtained. The analysis of the condensed solution by gas chromatography confirmed that 24.6 g of AOI-EG was obtained and the yield thereof was 73.3% (on the basis of 2-(2-aminoethoxy)ethanol). After 0.2 g of phenothiazine was added to the condensed solution, the pressure was reduced to 0.5 kPa with a vacuum pump, 1.7 g of an initial distillate was cut off, and 17.2 g of a main distillate was then obtained. The analysis of the main distillate by gas chromatography confirmed that the yield of AOI-EG was 48.9% (on the basis of 2-(2-aminoethoxy)ethanol).

### [Comparative Example 1]

### <Step (1)>

Into a 500-mL four-neck flask including an agitator, a thermometer, a dropping funnel and a reflux cooler, 20.0 g (0.19 mol) of 2-(2-aminoethoxy)ethanol and 200 mL of butyl acetate (in which the solubility of gaseous hydrogen chloride is 0.318 mole percent at 25°C and a partial pressure of 1 atm as given in *"*SOLUBILITY DATA SERIES Vol. 42 -HYDROGEN HALIDES IN NON-AQUEOUS SOLVENTS*-")* were charged under a nitrogen atmosphere. The flask was heated to 25°C, whereby 2-(2-aminoethoxy)ethanol was melted. Gaseous hydrogen chloride was supplied to the flask for one hour at a flow rate of 150 mL/min at a temperature of 75°C to 90°C.

### <Step (2)>

A reaction liquid obtained in Step (1) was heated to 95°C. To this reaction liquid, 0.2 g of phenothiazine was added and 21.9 g (0.21 mol) of methacryloyl chloride was then supplied in 1.0 hour, followed by heating at 95°C for 3.0 hours.

### <Step (3)>

A reaction liquid obtained in Step (2) was kept at 90°C. To this reaction liquid, 0.2 g of phenothiazine was added and 34.0 g (0.34 mol) of phosgene was then supplied in five hours, followed by heating at 90°C for one hour. The phosgene dissolved in this reaction liquid was removed by introducing nitrogen into this reaction liquid. The analysis of this resulting reaction liquid by gas chromatography confirmed that 14.9 g (0.075 mol) of MOI-EG was obtained and the yield thereof was 39.4% (on the basis of 2-(2-aminoethoxy)ethanol). The yield of a by-product formed by the addition of hydrogen chloride (HCl) to an unsaturated group in MOI-EG was 15.2% (on the basis of 2-(2-aminoethoxy)ethanol).

### [Example 6]

### <Step (1)>

Into a 500-mL four-neck flask including an agitator, a thermometer, a dropping funnel and a reflux cooler, 20.0 g (0.19 mol) of 2-(2-aminoethoxy)ethanol and 200 mL of 2,2,4-trimethylpentane (in which the solubility of gaseous hydrogen chloride is 0.0154 mole percent at 25°C and a partial pressure of 1 atm as given in "SOLUBILITY DATA SERIES Vol. 42 -HYDROGEN HALIDES IN NON-AQUEOUS SOLVENTS*-*") were charged under a nitrogen atmosphere. The flask was heated to 30°C, whereby 2-(2-aminoethoxy)ethanol was melted. Gaseous hydrogen chloride was supplied to the flask for one hour at a flow rate of 150 mL/min at a temperature of 75°C to 90°C.

### <Step (2)>

A reaction liquid obtained in Step (1) was heated to 95°C. To this reaction liquid, 0.2 g of phenothiazine was added and 21.9 g (0.21 mol) of methacryloyl chloride was then supplied in 1.0 hour, followed by heating at 95°C for 3.0 hours.

### <Step (3)>

A reaction liquid obtained in Step (2) was kept at 90°C. To this reaction liquid, 0.2 g of phenothiazine was added and 34.0 g (0.34 mol) of phosgene was then supplied in five hours, followed by heating at 90°C for one hour. The phosgene dissolved in this reaction liquid was removed by introducing nitrogen into this reaction liquid, whereby 167.4 g of a reaction liquid was obtained. This reaction liquid was kept stationary and thereby was separated into two layers. The analysis of each layer by gas chromatography confirmed that the yield of MOI-EG was 62.4% in total (on the basis of 2-(2-aminoethoxy)ethanol).

## Claims

1. A method for producing an ethylenically unsaturated group-containing isocyanate compound having an ether bond from an amino alcohol having an ether bond, the method comprising using a reaction solvent in which the solubility of hydrogen chloride is 0.1 mole percent or less at 25°C.

2. The method for producing an ethylenically unsaturated group-containing isocyanate compound having an ether bond according to Claim 1, wherein the reaction solvent is an aromatic or aliphatic hydrocarbon.

3. The method for producing an ethylenically unsaturated group-containing isocyanate compound having an ether bond according to Claim 1, wherein the reaction solvent is toluene.

4. The method for producing an ethylenically unsaturated group-containing isocyanate compound having an ether bond according to Claim 1, further comprising a reaction step performed at a temperature of 0°C to 100°C.

5. The method for producing an ethylenically unsaturated group-containing isocyanate compound having an ether bond according to Claim 4, wherein the reaction step includes:
Step (1) of reacting an amino alcohol (I) having an ether bond, represented by Formula (I) below, with hydrogen chloride to produce a compound (III) represented by Formula (III) below;
Step (2) of reacting the compound (III) with a compound (IV) represented by Formula (IV) or a compound (V) represented by Formula (V) to produce a compound (VI) represented by Formula (VI) below or a compound (VII) represented by Formula (VII) below;
Step (3) of reacting the compound (VI) or (VII) with phosgene to produce a compound (VIII) represented by Formula (VIII) below or a compound (II) represented by Formula (II) below; and
Step (4) of contacting the compound (VIII) or (II) with a basic nitrogen compound containing tertiary nitrogen: 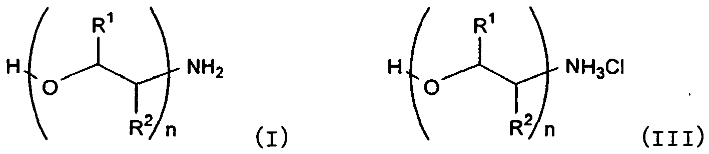 wherein R¹ and R² independently represent a hydrogen atom or a linear or branched alkyl group of 1 to 6 carbon atoms, and n represents an integer of 2 to 12; 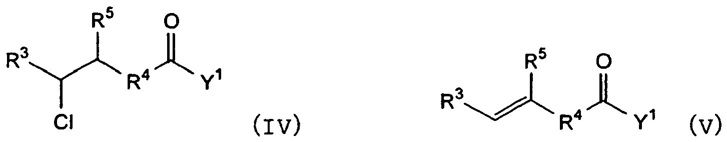 wherein R³ represents a hydrogen atom, a linear or branched alkyl of 1 to 6 carbon atoms, or an aryl group, R⁴ represents a single bond, or a linear or branched alkylene group of 1 to 5 carbon atoms, R⁵ represents a hydrogen atom or a methyl group, and Y¹ represents a hydroxy group, a chlorine atom or R⁶O- (where R⁶ represents an alkyl group of 1 to 6 carbon atoms); 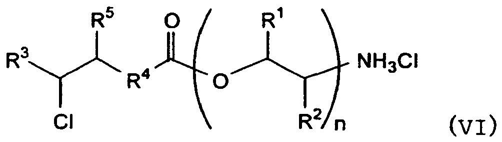 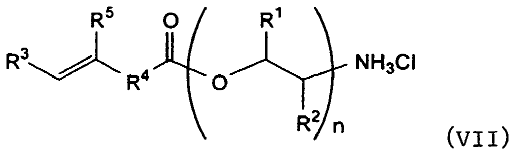 wherein R¹, R² and n are the same as R¹, R² and n, respectively, in Formula (I) and R³ to R⁵ are the same as _{R}³ to R⁵, respectively, in Formula (IV) or (V); 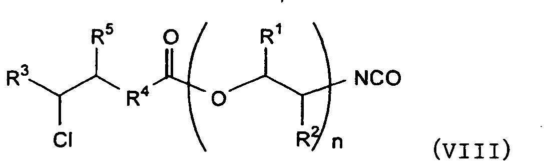 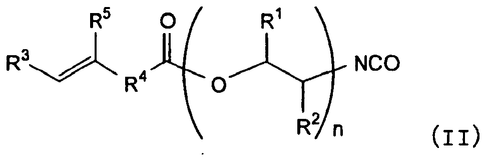 wherein R¹, R² and n are the same as R¹, R² and n, respectively in Formula (I); and R³ to R⁵ are the same as R³ to R⁵, respectively, in Formula (IV) or (V).

6. The method for producing an ethylenically unsaturated group-containing isocyanate compound having an ether bond according to Claim 5, further comprising a water-rinsing step of contacting a product obtained in Step (4) with water.

7. The method for producing an ethylenically unsaturated group-containing isocyanate compound having an ether bond according to Claim 5, wherein the reaction temperature of Step (2) is 65°C to 100°C.

8. The method for producing an ethylenically unsaturated group-containing isocyanate compound having an ether bond according to Claim 5, wherein Y¹ in the compound (IV) or (V) is a chlorine atom and the reaction of Step (2) is performed at reduced pressure.

9. The method for producing an ethylenically unsaturated group-containing isocyanate compound having an ether bond according to Claim 8, wherein the reaction of Step (2) is performed in such a manner that an inert gas is introduced into a reaction liquid.

10. The method for producing an ethylenically unsaturated group-containing isocyanate compound having an ether bond according to Claim 5, wherein the reaction of Step (3) is performed at reduced pressure.

11. The method for producing an ethylenically unsaturated group-containing isocyanate compound having an ether bond according to Claim 10, wherein the reaction of Step (3) is performed in such a manner that an inert gas is introduced into a reaction liquid.

## Patentansprüche

1. Verfahren zum Herstellen einer eine ethylenisch ungesättigte Gruppe enthaltenden Isocyanatverbindung mit einer Etherbindung aus einem Aminoalkohol mit einer Etherbindung, wobei das Verfahren die Verwendung eines Reaktionslösungsmittels umfasst, worin die Löslichkeit von Chlorwasserstoff 0,1 Mol-% oder weniger bei 25°C ist.

2. Verfahren zum Herstellen einer eine ethylenisch ungesättigte Gruppe enthaltenden Isocyanatverbindung mit einer Etherbindung nach Anspruch 1, wobei das Reaktionslösungsmittel ein aromatischer oder aliphatischer Kohlenwasserstoff ist.

3. Verfahren zum Herstellen einer eine ethylenisch ungesättigte Gruppe enthaltenden Isocyanatverbindung mit einer Etherbindung nach Anspruch 1, wobei das Reaktionslösungsmittel Toluol ist.

4. Verfahren zum Herstellen einer eine ethylenisch ungesättigte Gruppe enthaltenden Isocyanatverbindung mit einer Etherbindung nach Anspruch 1, das außerdem eine Reaktionsstufe umfasst, die bei einer Temperatur von 0 bis 100°C durchgeführt wird.

5. Verfahren zum Herstellen einer eine ethylenisch ungesättigte Gruppe enthaltenden Isocyanatverbindung mit einer Etherbindung nach Anspruch 4, wobei die Reaktionsstufe umfasst
eine Stufe (1), in der ein Aminoalkohol (I) mit einer Etherbindung der nachstehenden Formel (I) mit Chlorwasserstoff umgesetzt wird, wobei eine Verbindung (III) der nachstehenden Formel (III) hergestellt wird;
eine Stufe (2), in der die Verbindung (III) mit einer Verbindung (IV) der Formel (IV) oder einer Verbindung (V) der Formel (V) umgesetzt wird, wobei eine Verbindung (VI) der nachstehenden Formel (VI) oder eine Verbindung (VII) der nachstehenden Formel (VII) hergestellt wird;
eine Stufe (3), in der die Verbindung (VI) oder (VII) mit Phosgen umgesetzt wird, wobei eine Verbindung (VIII) der nachstehenden Formel (VIII) oder eine Verbindung (II) der nachstehenden Formel (II) hergestellte wird; und
eine Stufe (4), in der die Verbindung (VIII) oder (II) mit einer tertiären Stickstoff enthaltenden basischen Stickstoffverbindung kontaktiert wird: 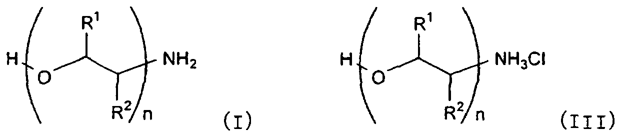 worin R¹ und R² unabhängig voneinander ein Wasserstoffatom oder eine lineare oder verzweigte Alkylgruppe mit 1 bis 6 Kohlenstoffatomen darstellen und n eine ganze Zahl von 2 bis 12 ist; 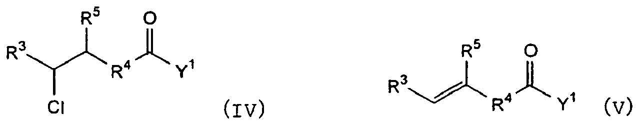 worin R³ ein Wasserstoffatom, eine lineare oder verzweigte Alkylgruppe mit 1 bis 6 Kohlenstoffatomen oder Arylgruppe darstellt, R⁴ eine Einfachbindung oder eine lineare oder verzweigte Alkylengruppe mit 1 bis 5 Kohlenstoffatomen darstellt, R⁵ ein Wasserstoffatom oder eine Methylgruppe darstellt und Y¹ eine Hydroxygruppe, ein Chloratom oder R⁶O- (worin R⁶ eine Alkylgruppe mit 1 bis 6 Kohlenstoffatomen darstellt) darstellt; 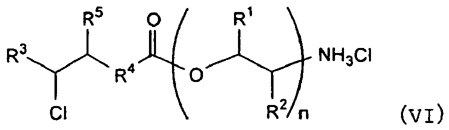 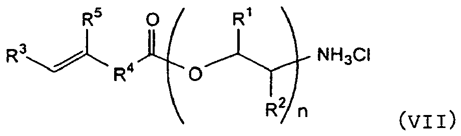 worin R¹, R² und n dieselbe Bedeutung haben wie R¹, R² bzw. n in der Formel (I) und R³ bis R⁵ dieselbe Bedeutung haben wie R³ bis R⁵ in der Formel (IV) oder (V); 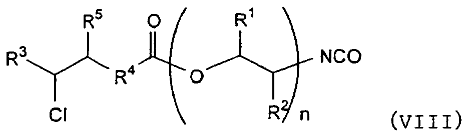 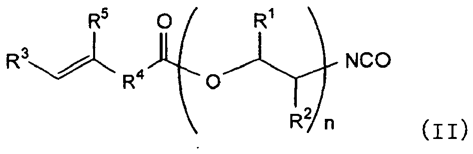 worin R¹, R² und n dieselbe Bedeutung haben wie R¹, R² bzw. n in der Formel (I); und R³ bis R⁵ dieselbe Bedeutung haben wie R³ bis R⁵ in der Formel (IV) oder (V).

6. Verfahren zum Herstellen einer eine ethylenisch ungesättigte Gruppe enthaltenden Isocyanatverbindung mit einer Etherbindung nach Anspruch 5, das außerdem eine Stufe zum Spülen mit Wasser umfasst, in der ein in der Stufe (IV) erhaltenes Produkt mit Wasser kontaktiert wird.

7. Verfahren zum Herstellen einer eine ethylenisch ungesättigte Gruppe enthaltenden Isocyanatverbindung mit einer Etherbindung nach Anspruch 5, wobei die Reaktionstemperatur der Stufe (2) 65°C bis 100°C ist.

8. Verfahren zum Herstellen einer eine ethylenisch ungesättigte Gruppe enthaltenden Isocyanatverbindung mit einer Etherbindung nach Anspruch 5, wobei Y¹ in der Verbindung (IV) oder (V) ein Chloratom ist und die Reaktion der Stufe (2) bei verringertem Druck durchgeführt wird.

9. Verfahren zum Herstellen einer eine ethylenisch ungesättigte Gruppe enthaltenden Isocyanatverbindung mit einer Etherbindung nach Anspruch 8, wobei die Reaktion der Stufe (2) auf eine solche Weise durchgeführt wird, dass ein Inertgas in die Reaktionsflüssigkeit eingeführt wird.

10. Verfahren zum Herstellen einer eine ethylenisch ungesättigte Gruppe enthaltenden Isocyanatverbindung mit einer Etherbindung nach Anspruch 5, wobei die Reaktion der Stufe (3) bei verringertem Druck durchgeführt wird.

11. Verfahren zum Herstellen einer eine ethylenisch ungesättigte Gruppe enthaltenden Isocyanatverbindung mit einer Etherbindung mit einer Etherbindung nach Anspruch 10, wobei die Reaktion der Stufe (3) auf eine solche Weise durchgeführt wird, dass ein Inertgas in eine Reaktionsflüssigkeit eingeführt wird.

## Revendications

1. Procédé de production d'un composé isocyanate contenant un groupe éthyléniquement insaturé présentant une liaison éther à partir d'un amino alcool présentant une liaison éther, le procédé comprenant l'utilisation d'un solvant de réaction dans lequel la solubilité du chlorure d'hydrogène est de 0,1 pourcent en mole ou inférieure à 25°C.

2. Procédé de production d'un composé isocyanate contenant un groupe éthyléniquement insaturé présentant une liaison éther selon la revendication 1, dans lequel le solvant de réaction est un hydrocarbure aromatique ou aliphatique.

3. Procédé de production d'un composé isocyanate contenant un groupe éthyléniquement insaturé présentant une liaison éther selon la revendication 1, dans lequel le solvant de réaction est le toluène.

4. Procédé de production d'un composé isocyanate contenant un groupe éthyléniquement insaturé présentant une liaison éther selon la revendication 1, comprenant de plus une étape de réaction réalisée à une température de 0°C à 100°C.

5. Procédé de production d'un composé isocyanate contenant un groupe éthyléniquement insaturé présentant une liaison éther selon la revendication 4, dans lequel l'étape de réaction comprend :
l'étape (1) de réaction d'un amino alcool (I) présentant une liaison éther, représenté par la formule (I) ci-dessous, avec du chlorure d'hydrogène pour produire un composé (III) représenté par la formule (III) ci-dessous ;
l'étape (2) de réaction du composé (III) avec un composé (IV) représenté par la formule (IV) ou un composé (V) représenté par la formule (V) pour produire un composé (VI) représenté par la formule (VI) ci-dessous ou un composé (VII) représenté par la formule (VII) ci-dessous ;
l'étape (3) de réaction du composé (VI) ou (VII) avec du phosgène pour produire un composé (VIII) représenté par la formule (VIII) ci-dessous ou un composé (II) représenté par la formule (II) ci-dessous ; et
l'étape (4) de mise en contact du composé (VIII) ou (II) avec un composé d'azote basique contenant de l'azote tertiaire : 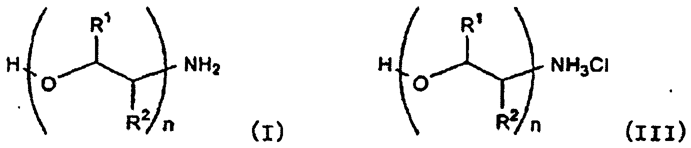 où R¹ et R² représentent indépendamment un atome d'hydrogène ou un groupe alkyle linéaire ou ramifié de 1 à 6 atomes de carbone, et n représente un nombre entier de 2 à 12 ; 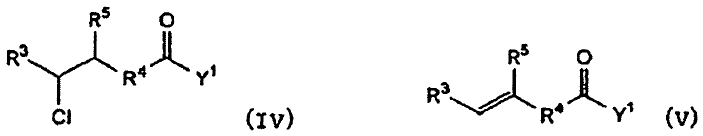 où R³ représente un atome d'hydrogène, un groupe alkyle linéaire ou ramifié de 1 à 6 atomes de carbone, ou un groupe aryle, R⁴ représente une liaison simple, ou un groupe alkylène linéaire ou ramifié de 1 à 5 atomes de carbone, R⁵ représente un atome d'hydrogène ou un groupe méthyle, et Y¹ représente un groupe hydroxy, un atome de chlore ou R⁶O-(où R⁶ représente un groupe alkyle de 1 à 6 atomes de carbone) ; 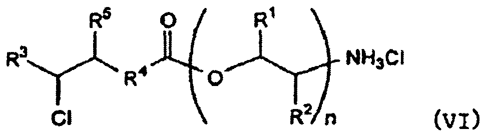 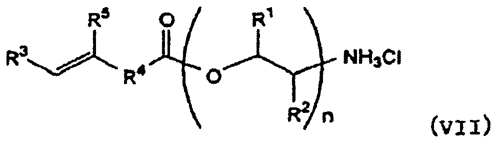 où R¹, R² et n sont identiques à R¹, R² et n, respectivement, dans la formule (I) et R³ à R⁵ sont identiques à R³ à R⁵, respectivement, dans la formule (IV) ou (V) ; 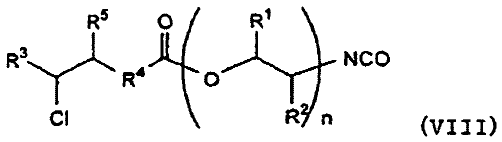 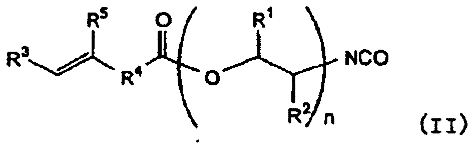 où R¹, R² et n sont identiques à R¹, R² et n, respectivement dans la formule (I) ; et R³ à R⁵ sont identiques à R³ à R⁵, respectivement, dans la formule (IV) ou (V).

6. Procédé de production d'un composé isocyanate contenant un groupe éthyléniquement insaturé présentant une liaison éther selon la revendication 5, comprenant de plus une étape de rinçage à l'eau consistant à mettre un produit obtenu dans l'étape (4) en contact avec de l'eau.

7. Procédé de production d'un composé isocyanate contenant un groupe éthyléniquement insaturé présentant une liaison éther selon la revendication 5, dans lequel la température de réaction de l'étape (2) est de 65°C à 100°C.

8. Procédé de production d'un composé isocyanate contenant un groupe éthyléniquement insaturé présentant une liaison éther selon la revendication 5, dans lequel Y¹ dans le composé (IV) ou (V) est un atome de chlore et la réaction de l'étape (2) est réalisée à pression réduite.

9. Procédé de production d'un composé isocyanate contenant un groupe éthyléniquement insaturé présentant une liaison éther selon la revendication 8, dans lequel la réaction de l'étape (2) est réalisée de telle manière qu'un gaz inerte est introduit dans un liquide de réaction.

10. Procédé de production d'un composé isocyanate contenant un groupe éthyléniquement insaturé présentant une liaison éther selon la revendication 5, dans lequel la réaction de l'étape (3) est réalisée à pression réduite.

11. Procédé de production d'un composé isocyanate contenant un groupe éthyléniquement insaturé présentant une liaison éther selon la revendication 10, dans lequel la réaction de l'étape (3) est réalisée de telle manière qu'un gaz inerte est introduit dans un liquide de réaction.
